# EUROPEAN PATENT SPECIFICATION

(11) **EP 1 734 871 B1**
(45) Date of publication and mention of the grant of the patent: **01.07.2009**
(21) Application number: 05734962.3
(22) Date of filing: 05.04.2005
(51) Int. Cl.: A61B 17/06, A61B 17/00, A61F 5/00

(54) **MEDICAL DEVICE FOR PROBING AND CARRYING OUT A CONCOMITANT ELASTIC TRACTION OF A FISTULA, MORE PARTICULARLY PERIANAL FISTULAS**
MEDIZINPRODUKT FÜR DIE UNTERSUCHUNG UND AUSÜBUNG EINES GLEICHZEITIGEN ELASTISCHEN ZUGS AUF EINE FISTEL, INSBESONDERE EINE PERIANALE FISTEL
DISPOSITIF MEDICAL DE SONDAGE ET D'EXECUTION D'UNE TRACTION ELASTIQUE CONCOMITANTE D'UNE FISTULE, PLUS PARTICULIEREMENT DE FISTULES PERIANALES

(30) Priority: 08.04.2004 IT CE20040002
(43) Date of publication of application: 27.12.2006
(73) Proprietor: Antropoli, Carmine, 81020 Sant'Angelo in Formis, Capua CE (IT)
(72) Inventor: Antropoli, Carmine, 81020 Sant'Angelo in Formis, Capua CE (IT)
(74) Representative: Sarpi, Maurizio
(86) International application number: PCT/IT2005/000185
(87) International publication number: WO 2005/096957

(56) References cited:
- ES-A1- 2 116 914
- US-A- 454 327
- PATENT ABSTRACTS OF JAPAN vol. 2003, no. 11, 5 November 2003 (2003-11-05) & JP 2003 210502 A (YUFU ITONAGA CO LTD), 29 July 2003 (2003-07-29)

## Description

US-A-454327 discloses a fistula-ligature applicator comprising a flexible thread having a tip, said thread having a fore end portion covered by a stiffening jacket which can be removed by sliding on the thread and leaves only said fore end portion uncovered.

The present invention relates to a medical device for probing fistulas, more particularly perianal fistulas, and carrying out a concomitant elastic traction to such fistulas by inserting a ligature therein.

More specifically, such medical device consists of a thin, flexible ligature having a fore end portion covered by a removable tubular jacket leaving only the tip uncovered, and a stiffened rear end portion. To tie a fistula, the tip is provided with a through hole into which the rear end portion is inserted.

Probing perianal fistulas is carried out by special probes or button-headed needles that do not allow, however, also the head of the ligature to pass through the fistula at the same time.

Therefore, a drawback of the state of art is that the conventional probing means inserts a ligature into the fistula only at a second time and with great difficulty.

A second drawback is that the ligature could break upon passing through the fistula, thus causing the operation to become more complicated and longer as well.

A third drawback is that the ligature could be inserted wrongly.

The main object of the present invention is to overcome such problems by providing a disposable medical device that allows perianal and non-perianal fistulas to be probed and at the same time a ligature for the elastic traction of fistulas to be inserted. This has been accomplished according to the present invention by providing a disposable medical device generally having the shape of a probe and consisting of a thread with a fore end portion covered by a removable tubular jacket, and a stiffened rear end portion.

According to a feature of the invention, such stiffened removable tubular jacket leaves the fore end of the thread uncovered. Formed into such fore end is a through hole into which the stiffened rear end portion of the thread is inserted to pass with its tip through the fistula and coming out to the anal side to be blocked after having removed the tubular jacket.

A better understanding of the finding will follow from the following detailed description with reference to the accompanying drawings that show two preferred embodiments only by way of example.

In the drawings:
Figure 1 is a longitudinal section of a first embodiment of the disposable medical device;
Figure 2 is an enlarged detail of the forward end of the finding;
Figure 3 is a partial side view of the finding;
Figure 4 shows the insertion of the finding into a fistula;
Figure 5 shows the fore end portion of the finding coming out to the anal side;
Figure 6 shows the finding after the tubular jacket has been removed;
Figure 7 shows the fistula with ligature;
Figure 8A shows a second embodiment of the finding;
Figure 8B is an enlarged detail of Figure 8A;
Figures 9A and 9B show the tip of the finding provided with a hole;

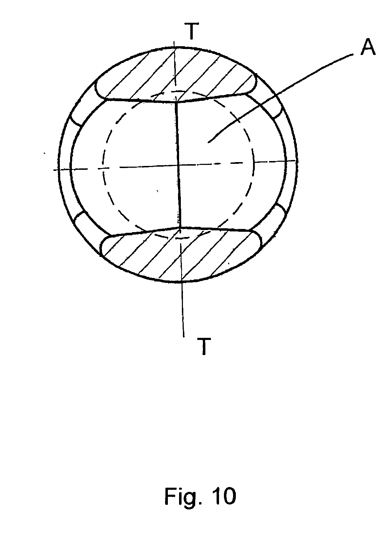

Figure 10 shows the section of the hole in the tip of the preceding figure taken along the line T-T;
Figure 11 shows a support of the tip of figures 9A and 9B;
Figure 12 shows a fitting for syringe to be applied to the finding.

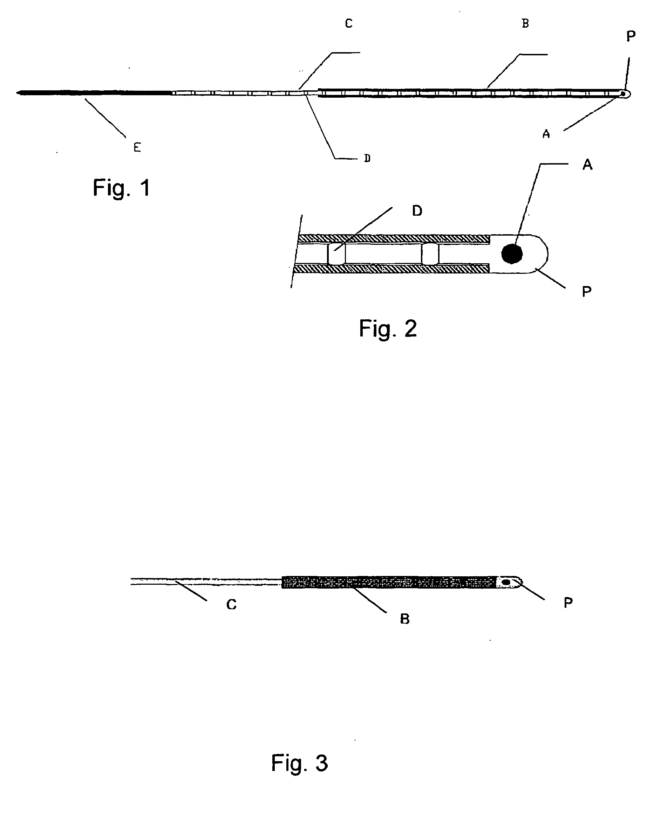

With reference to figures 1, 2 and 3, the disposable medical device of the disclosed embodiment includes a flexible thread C of silicone having a fore end portion capable of probing fistulas and covered and stiffened by a tubular jacket B which can be removed by sliding on the thread. Such tubular jacket B is positioned adjacent the fore tip P of thread C having a diameter greater than thread C and a blunted shape, and is provided with a transversal hole A which allows the rear end portion E to be passed therethrough. Advantageously, such end portion E is suitably stiffened in order to help the insertion into hole A having a diameter greater than or equal to the diameter of the flexible thread and its rear end E. Bulges or fastening nodes D are distributed generally uniformly on the thread which allow the physician to grasp the fistula which is tied by providing a more or less elastic traction.

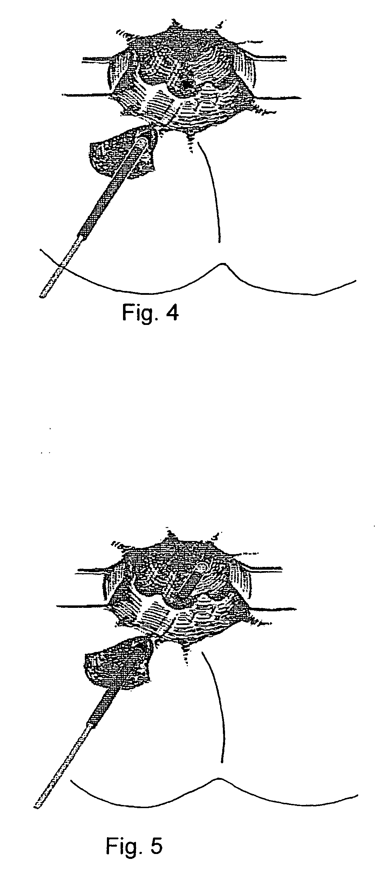

In operation, once the fistular orifice is located, a probing is carried out by a blunted fore tip P of thread C (figure 4) which passes through such orifice and comes out to the anal side (figure 5).

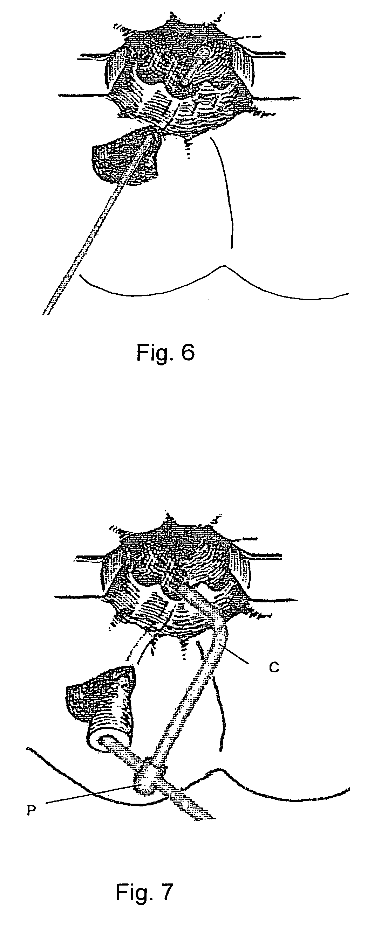

By holding the fore tip P of thread C by the fingers or a surgical instrument to position thread C, the tubular stiffening jacket B is taken off so as to leave only thread C in the fistula (figure 6). Once tubular jacket B is taken off, the stiffened rear portion E is inserted into hole A of tip P (figure 7): fistula is thugs probed and tied by thread C.

The thus tied fistula is grasped on the discretion of the physician according to requirements by fastening nodes D having such a diameter as to interfere with the diameter of hole A, thus preventing thread C from sliding inside the latter and allowing an operator to pull thread C to a suitable extent. The second embodiment provides in combination a thread C, a tip P, and a suitable support S able to receive such tip.

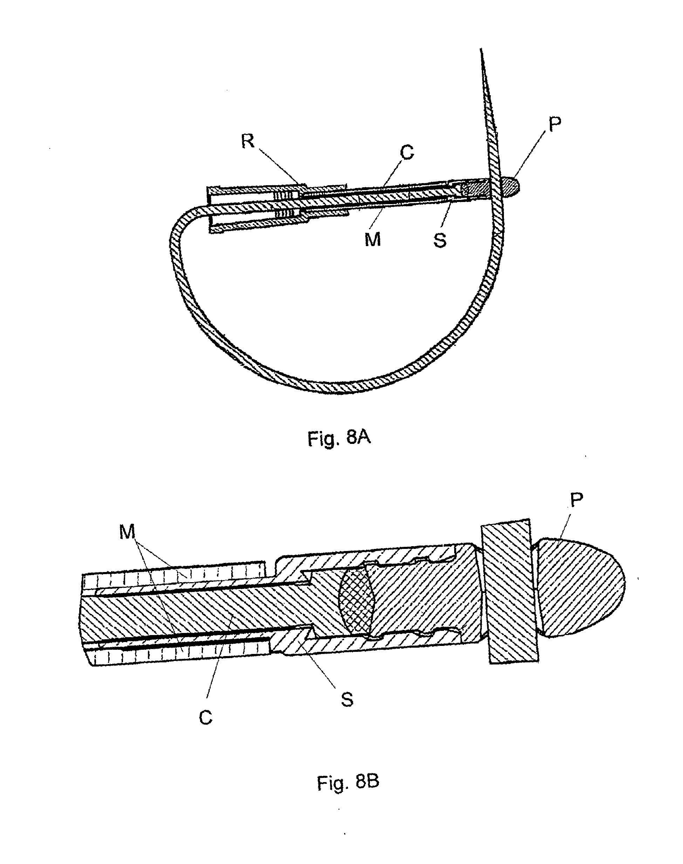

Tip P is forced into support S which is in turn forced onto thread C (figure 8).

Thread C is partially covered by a tubular jacket B which can be removed by sliding thereon and has a stiffened rear end portion E.

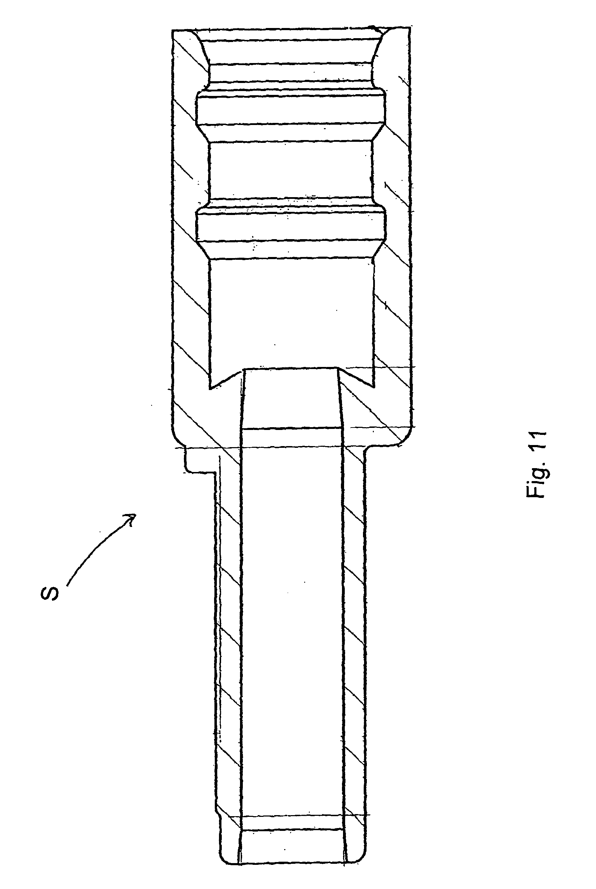

Support S includes a rear portion able to receive a portion of thread C, and a fore portion with larger diameter able to receive tip P and provided with two inner annular grooves (figure 11).

Once support S is inserted onto thread C, it is secured to the thread by means able to lock it firmly. In the disclosed example such means consists of a sleeve M of rigid plastic material which holds support S together with thread C (figures 8A and 8B).

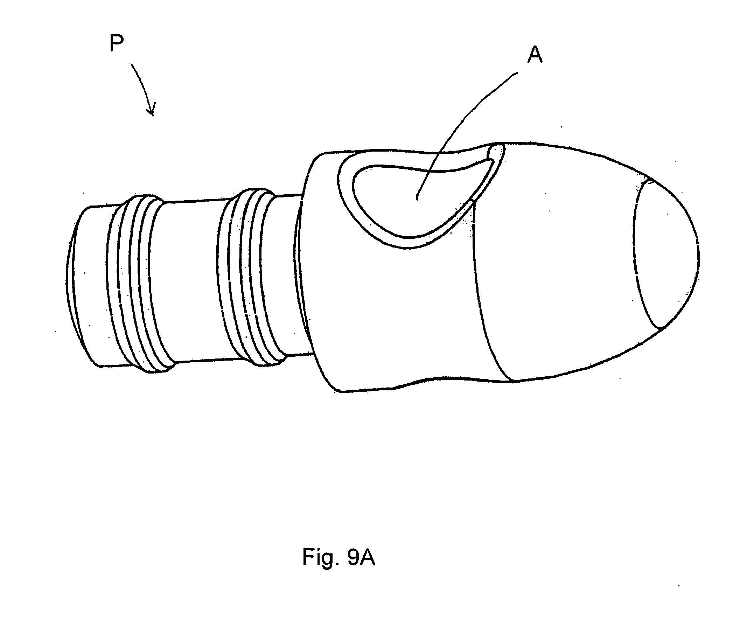

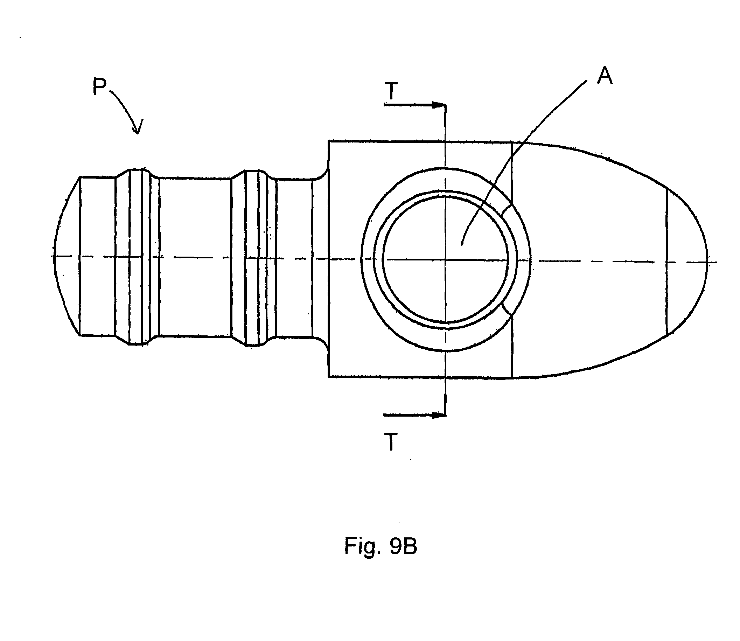

Tip P includes a rear portion provided with two annular bulges, and a fore portion with larger diameter having a blunted shape and provided with a transversal hole A (figures 9A and 9B).

Once such tip P is inserted into support S, the annular bulges of the rear portion of tip P are fitted into the corresponding annular grooves of the fore portion of support S, the fore portion of tip P projecting from support S.

According to a further feature of the invention, transversal through hole A of tip P is tapered from both outer sides to the centre and has an inner central diameter which is slightly lower than the diameter of thread C such that the sliding of thread C inside the hole is hindered and an operator should apply a suitable force of traction to pull thread C. Instead of silicone thread C can be made of latex, rubber, polyester, silk, or other similar materials or even reabsorbable materials of the known type. Removable tubular stiffening jacket B can be made of a suitable multilayer material, the inner and outer layers being self-lubricated to facilitate the sliding of jacket B on thread C and the insertion into fistular orifice.

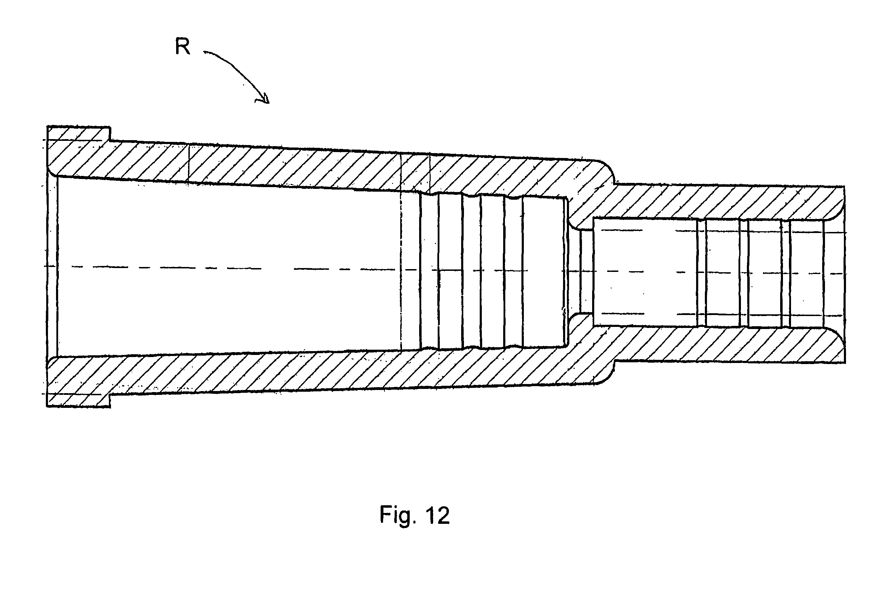

Advantageously, to put dyes into the human body thread C is provided with a fitting R for syringe which can be removed by sliding on the thread (figure 12).

A second advantage is that the mode of use is very easy to learn in a short time.

A third advantage is the precision of such medical device.

Of course, the device has several geometrical shapes, different size and length.

The present invention has been described and illustrated according to some preferred embodiments and a variation thereof, however, it is self-evident that anyone skilled in the art can make technically equivalent modifications and/or replacements without departing from the scope of the claims.

## Claims

1. A disposable medical device, whereby
in order to carry out at the same time probing of fistulas and insertion of a ligature for the elastic traction of fistulas comprising a flexible thread (C) having a blunted tip (P), said thread being provided with a fore end portion covered by a tubular stiffening jacket which can be removed by sliding on the thread and leaves only said fore tip (P) uncovered, said tip (P) being provided with a through hole (A) into which the stiffened rear end portion (E) of thread (C) is inserted.

2. The medical device according to claim 1, **characterized in that** thread (C) is provided with bulges or fastening nodes (D) having a diameter larger than the hole (A) such that the sliding of thread (C) inside the hole is hindered and an operator should apply a suitable force of traction to pull thread (C).

3. The medical device according to the preceding claim, **characterized in that** said fastening nodes (D) are distributed generally uniformly on the thread.

4. The medical device according to claim 1, **characterized in that** fore tip (P) of thread (C) has a larger diameter than the thread.

5. The medical device according to claim 1, **characterized in that** stiffening tubular jacket (B) is positioned adjacent said fore tip (P).

6. The medical device according to claim 1, **characterized in that** hole (A) of thread (C) is tapered from both outer sides to the centre and has an inner central diameter which is slightly lower than the diameter of thread (C) such that the sliding of thread (C) inside the hole is hindered and an operator should apply a suitable force of traction to pull said thread (C).

7. The medical device according to claim 1, **characterized in that** fore tip (P) is forced into a support (S) which is in turn forced onto thread (C).

8. The medical device according to the preceding claim, **characterized in that** support (S) includes a rear portion able to receive the fore end portion of thread (C), and a fore portion with larger diameter able to receive tip (P) and provided with at least one inner annular groove, said support (S) being secured to thread (C) by means of the known type.

9. The medical device according to claim 8, **characterized in that** fore tip (P) includes a rear portion provided with at least one annular bulge able to be inserted into the corresponding annular groove of support (S).

10. The medical device according to any preceding claim, **characterized in that** once fore tip (P) of thread (C) comes out to the anal side after having passed the fistula, tubular jacket (B) is removed to allow the tread to be folded on itself to perform the ligature of the fistula.

11. The medical device according to any preceding claim, **characterized in that** thread (C) is provided with a fitting (R) for syringe which is removable by sliding on the thread.

12. The medical device according to any preceding claim, **characterized in that** thread (C) is made of silicone, or latex, or polyester, or silk, or other similar materials.

13. The medical device according to any claim 1 to 11, **characterized in that** thread (C) is made of reabsorbable material of the known type.

14. The medical device according to any preceding claim, **characterized in that** stiffening tubular jacket (B) is made of plastic material.

15. The medical device according to the preceding claim, **characterized in that** said plastic material has multiple layers, both inner and outer layers being self-lubricated to help the sliding of jacket (B) on thread (C) and the insertion into fistular orifice.

## Patentansprüche

1. Medizinische Einwegvorrrichtung bzw. Einwegmedizinprodukt, wobei diese/s, zum gleichzeitigen Ausführen der Untersuchung von Fisteln und dem Einführen einer Ligatur für den elastischen Zug auf die Fisteln, einen biegsamen Faden bzw. ein Garn (C) mit einer abgestumpften Spitze umfasst, wobei der Faden mit einem vorderen Endabschnitt versehen ist, der mit einer rohr- bzw. schlauchförmigen Versteifungshülse bedeckt ist, die durch Gleiten bzw. Schieben auf dem Faden entfernt werden kann und nur die vordere Spitze (P) unbedeckt lässt, wobei die Spitze (P) mit einer Durchgangsbohrung bzw. durchgehenden Öffnung (A) versehen ist, in die der versteifte hintere Endabschnitt (E) des Fadens (C) eingeführt ist.

2. Medizinische Vorrichtung nach Anspruch 1, **dadurch gekennzeichnet, dass** der Faden (C) mit Auswölbungen oder Befestigungsknoten (D) versehen ist, die einen Durchmesser größer als das Loch (A) haben, so dass das Schieben des Fadens in dem Loch erschwert bzw. behindert wird, und ein Operateur bzw. eine den Eingriff vornehmende Person eine geeignete Zugkraft ausüben sollte, um den Faden (C) zu ziehen.

3. Medizinische Vorrichtung nach dem vorhergehenden Anspruch, **dadurch gekennzeichnet, dass** die Befestigungsknoten (D) im Allgemeinen gleichförmig auf dem Faden verteilt sind.

4. Medizinische Vorrichtung nach Anspruch 1, **dadurch gekennzeichnet, dass** die vordere Spitze (P) des Fadens (C) einen größeren Durchmesser als der Faden hat.

5. Medizinische Vorrichtung nach Anspruch 1, **dadurch gekennzeichnet, dass** die versteifende rohrförmige Hülse (B) an die vordere Spitze (P) angrenzend angeordnet ist.

6. Medizinische Vorrichtung nach Anspruch 1, **dadurch gekennzeichnet, dass** sich das Loch (A) des Fadens (C) von beiden Außenseiten zur Mitte hin verjüngt und einen inneren mittleren Durchmesser hat, der etwas unterhalb des Durchmessers des Fadens (C) liegt, so dass das Schieben des Fadens (C) in dem Loch erschwert wird, und ein Anwender geeignete Zugkraft ausüben sollte, um den Faden (C) zu ziehen.

7. Medizinische Vorrichtung nach Anspruch 1, **dadurch gekennzeichnet, dass** die vordere Spitze (P) in eine Stützvorrichtung bzw. Halterung (S) gedrückt wird, die wiederum auf den Faden (C) gedrückt wird.

8. Medizinische Vorrichtung nach dem vorhergehenden Anspruch, **dadurch gekennzeichnet, dass** die Stützvorrichtung (S) einen hinteren Abschnitt enthält, der den vorderen Endabschnitt des Fadens (C) aufnehmen kann, und einen vorderen Abschnitt mit größerem Durchmesser, der die Spitze (P) aufnehmen kann und mit wenigstens einer inneren ringförmigen Nut versehen ist, wobei die Stützvorrichtung (S) an dem Faden (C) mit bekannten Mitteln gesichert ist.

9. Medizinische Vorrichtung nach Anspruch 8, **dadurch gekennzeichnet, dass** die vordere Spitze (P) einen hinteren Abschnitt enthält, der mit wenigstens einer ringförmigen Wulst bzw. Auswölbung versehen ist, die in die entsprechende ringförmige Nut der Stützvorrichtung (S) eingeführt werden kann.

10. Medizinische Vorrichtung nach einem vorherigen Anspruch, **dadurch gekennzeichnet, dass** sobald die vordere Spitze (P) des Fadens (C) auf der annalen Seite, nachdem sie die Fistel durchdrungen hat, austritt, die rohrförmige Hülse (B) entfernt wird, damit der Faden sich auf bzw. um sich selbst wickeln kann, um die Ligatur der Fistel auszuführen.

11. Medizinische Vorrichtung nach einem vorhergehenden Anspruch, **dadurch gekennzeichnet, dass** der Faden (C) mit einem Passstück bzw. Anschlussteil (R) für eine Spritze versehen ist, das durch Schieben auf dem Faden entfernbar ist.

12. Medizinische Vorrichtung nach einem vorhergehenden Anspruch, **dadurch gekennzeichnet, dass** der Faden (C) aus Silicon oder Latex oder Polyester oder Seide oder anderen ähnlichen Materialien besteht.

13. Medizinische Vorrichtung nach einem der Ansprüche 1 bis 11, **dadurch** gekenntzeichnet, dass der Faden (C) aus einem resorbierbaren Material bekannter Art hergestellt ist.

14. Medizinische Vorrichtung nach einem vorhergehenden Anspruch, **dadurch gekennzeichnet, dass** die versteifende rohrförmige Hülse (B) aus Kunststoffmaterial hergestellt ist.

15. Medizinische Vorrichtung nach dem vorhergehenden Anspruch, **dadurch gekennzeichnet, dass** das Kunststoffmaterial mehrere Schichten aufweist, wobei sowohl die inneren als auch äußeren Schichten selbstschmierend sind, um das Gleiten der Hülse (B) auf dem Faden (C) und die Einführung in die fistelartige Öffnung zu erleichtern.

## Revendications

1. Dispositif médical jetable, destiné à effectuer en même temps un sondage de fistules et une insertion d'une ligature pour une traction élastique de fistules comprenant un fil souple (C) qui présente un bout émoussé (P), ledit fil étant doté d'une partie d'extrémité antérieure couverte par une enveloppe de raidissement tubulaire qui peut être retirée en la faisant glisser sur le fil et qui laisse seulement ledit bout antérieur (P) découvert, ledit bout (P) étant doté d'un trou traversant (A) dans lequel la partie d'extrémité arrière raidie (E) du fil (C) est insérée.

2. Dispositif médical selon la revendication 1, **caractérisé en ce que** le fil (C) est doté de renflements ou de noeuds de fixation (D) qui présentent un diamètre plus grand que le trou (A) de telle sorte que le glissement du fil (C) à l'intérieur du trou soit entravé et qu'un opérateur soit obligé d'appliquer une force de traction appropriée pour tirer le fil (C).

3. Dispositif médical selon la revendication précédente, **caractérisé en ce que** lesdits noeuds de fixation (D) sont répartis en général de manière uniforme sur le fil.

4. Dispositif médical selon la revendication 1, **caractérisé en ce que** le bout antérieur (P) du fil (C) présente un diamètre plus grand que le fil.

5. Dispositif médical selon la revendication 1, **caractérisé en ce que** l'enveloppe tabulaire de raidissement (B) est positionnée adjacente audit bout antérieur (P).

6. Dispositif médical selon la revendication 1, **caractérisé en ce que** le trou (A) du fil (C) est conique à partir des deux côtés extérieurs vers le centre et présente un diamètre central intérieur qui est légèrement inférieur au diamètre du fil (C) de telle sorte que le glissement du fil (C) à l'intérieur du trou soit entravé et qu'un opérateur soit obligé d'appliquer une force de traction appropriée pour tirer ledit fil (C).

7. Dispositif médical selon la revendication 1, **caractérisé en ce que** le bout antérieur (P) est entré de force dans un support (S) qui est à son tour entré de force sur le fil (C).

8. Dispositif médical selon la revendication précédente, **caractérisé en ce que** le support (S) inclut une partie arrière capable de recevoir la partie d'extrémité antérieure du fil (C), et une partie antérieure qui présente un diamètre plus grand capable de recevoir le bout (P) et doté d'au moins une rainure annulaire intérieure, ledit support (S) étant fixé sur le fil (C) à l'aide de moyens d'un type connu.

9. Dispositif médical selon la revendication 8, **caractérisé en ce que** le bout antérieur (P) inclut une partie arrière dotée d'au moins un renflement annulaire capable d'être inséré dans la rainure annulaire correspondante du support (S).

10. Dispositif médical selon l'une quelconque des revendications précédentes, **caractérisé en ce qu'**une fois que le bout antérieur (P) du fil (C) est sorti du côté anal après avoir passé la fistules, l'enveloppe tubulaire (B) est retirée de manière à permettre de replier le fil sur lui-même de façon à exécuter la ligature de la fistule.

11. Dispositif médical selon l'une quelconque des revendications précédentes, **caractérisé en ce que** le fil (C) est doté d'un raccord (R) destiné à une seringue, qui peut être retiré par un coulissement sur le fil.

12. Dispositif médical selon l'une quelconque des revendications précédentes, **caractérisé en ce que** le fil (C) est réalisé en silicone, en latex, en polyester ou en soie, ou dans d'autres matériaux similaires.

13. Dispositif médical selon l'une quelconque des revendications 1 à 11, **caractérisé en ce que** le fil (C) est réalisé dans un matériau d'un type connut qui peut être résorbé.

14. Dispositif médical selon l'une quelconque des revendications précédentes, **caractérisé en ce que** l'enveloppe tubulaire de raidissement (B) est réalisée dans un matériau de matière plastique.

15. Dispositif médical selon l'une quelconque des revendications précédentes, **caractérisé en ce que** ledit matériau de matière plastique présente de multiples couches intérieures et extérieures auto-lubrifiées de manière à faciliter le glissement de l'enveloppe (B) sur le fil (C) et l'insertion dans l'orifice fistulaire.
